# EUROPEAN PATENT APPLICATION

(11) **EP 4 458 255 A1**
(43) Date of publication of application: **06.11.2024**
(21) Application number: 23759582.2
(22) Date of filing: 27.01.2023
(51) Int. Cl.: A61B 5/00, A61B 5/0507

(54) **SIGHTING DEVICE AND MEASUREMENT DEVICE**

(30) Priority: 24.02.2022 JP 2022027040
(71) Applicant: OMRON Corporation, Kyoto 600-8530 (JP)
(72) Inventor: OZAWA, Hisashi, Kyoto-shi, Kyoto 600-8530 (JP); SAITO, Keisuke, Kyoto-shi, Kyoto 600-8530 (JP); YAWATA, Masaki, Kyoto-shi, Kyoto 600-8530 (JP)
(74) Representative: Isarpatent
(86) International application number: PCT/JP2023/002655
(87) International publication number: WO 2023/162576

(57) **Abstract**

A sighting device is a sighting device used in a measuring apparatus including a signal detector that detects a signal related to biological information reflected from a person to be measured, the sighting device including: a visible indicator; and a viewing range specifier that specifies a viewing range of the indicator. A relative position between the indicator and the viewing range specifier is determined such that the viewing range of the indicator specified by the viewing range specifier forms an area included in a detection range of the signal by the signal detector.

## Description

### TECHNICAL FIELD

The present invention relates to a technique for improving the measurement accuracy of biological information.

### BACKGROUND ART

Techniques for acquiring biological information of a person to be measured with non-contact unit such as various sensors and radars have been put into practical use. For example, with a measuring apparatus according to Patent Document 1, a technique for improving the measurement accuracy of biological information of a person to be measured by irradiating a detection range of a sensor unit, which detects a microwave irradiated to the person to be measured, with a pointer, has been proposed.

### PRIOR ART DOCUMENT

### PATENT DOCUMENT

Patent Document 1: Japanese Unexamined Patent Publication No. 2017-029452

### SUMMARY OF THE INVENTION

### PROBLEMS TO BE SOLVED BY THE INVENTION

However, in the prior art, when the measuring apparatus is installed in a relatively bright place, it is difficult to view the pointer used for irradiation, and there is a possibility that the measurement accuracy of the biological information decreases.

The present invention has been made in view of the above circumstances, and provides a technique for improving the measurement accuracy of biological information of a person to be measured.

### MEANS FOR SOLVING THE PROBLEM

In order to achieve the above object, the present invention adopts the following configurations.

One aspect of the present invention is a sighting device used in a measuring apparatus including a signal detector that detects a signal related to biological information reflected from a person to be measured, the sighting device including: a visible indicator; and a viewing range specifier that specifies a viewing range of the indicator. A relative position between the indicator and the viewing range specifier is determined such that the viewing range of the indicator specified by the viewing range specifier forms an area included in a detection range of the signal by the signal detector. As a result, when the indicator is visible within the range specified by the viewing range specifier, it is possible to confirm that the person to be measured is within the signal detection range of the signal detector. Therefore, it is possible to more reliably detect the signal by the signal detector and improve the measurement accuracy of the measuring apparatus.

The indicator may be formed on an inner surface of a recess formed in an exterior member of the measuring apparatus, and the viewing range specifier is an opening of the recess. The sighting device may further include two walls facing each other. The indicator may be formed on one wall of the two walls, and the viewing range specifier may be a window portion penetrating another wall of the two walls. The viewing range specifier may be two rod members extending from an outer surface of an exterior member of the measuring apparatus, the two rod members being disposed at positions for specifying a viewing range of the indicator in at least one of an azimuth angle direction or an elevation angle direction as viewed from the signal detector. The viewing range specifier may be two indicators visible from the outside of the measuring apparatus, the two indicators being disposed at positions for specifying a viewing range of the indicator in an azimuth angle direction or an elevation angle direction as viewed from the signal detector. As a result, a user of the measuring apparatus or a person to be measured can specify the signal detection range of the signal detector with a simple configuration. Moreover, one aspect of the present invention is a measuring apparatus including the sighting device and the signal detector. The measuring apparatus may further include an adjuster configured to change a direction of the sighting device in at least one of an azimuth angle direction or an elevation angle direction as viewed from the signal detector.

### EFFECT OF THE INVENTION

According to the present invention, it is possible to improve the measurement accuracy of biological information of a person to be measured.

### BRIEF DESCRIPTION OF THE DRAWINGS

Fig. 1 is a view illustrating a schematic configuration of a measuring apparatus according to an embodiment.
Fig. 2A is a top view of the measuring apparatus according to the embodiment, and Fig. 2B is a side view of the measuring apparatus.
Figs. 3A and 3B are views illustrating a relationship between a signal detection range of an antenna and a visible range of a mark of a sighting device in the measuring apparatus according to the embodiment.
Fig. 4 is a view illustrating a schematic configuration of a measuring apparatus according to an embodiment.
Fig. 5 is a view illustrating a schematic configuration of a measuring apparatus according to a modification.
Fig. 6 is a view illustrating a schematic configuration of a measuring apparatus according to a modification.
Fig. 7 is a view illustrating a schematic configuration of a measuring apparatus according to a modification.
Fig. 8 is a view illustrating a schematic configuration of a measuring apparatus according to a modification.
Fig. 9 is a view illustrating a schematic configuration of a measuring apparatus according to a modification.
Fig. 10 is a view illustrating a schematic configuration of a measuring apparatus according to a modification.

### MODE FOR CARRYING OUT THE INVENTION

### <Application Example>

An application example of the present invention will be described. In the prior art, since the visibility of the pointer indicating the microwave irradiation range depends on the environment (brightness, etc.) in which the measuring apparatus is installed, the measurement accuracy of the biological information of the person to be measured may decrease depending on the installation environment of the measuring apparatus.

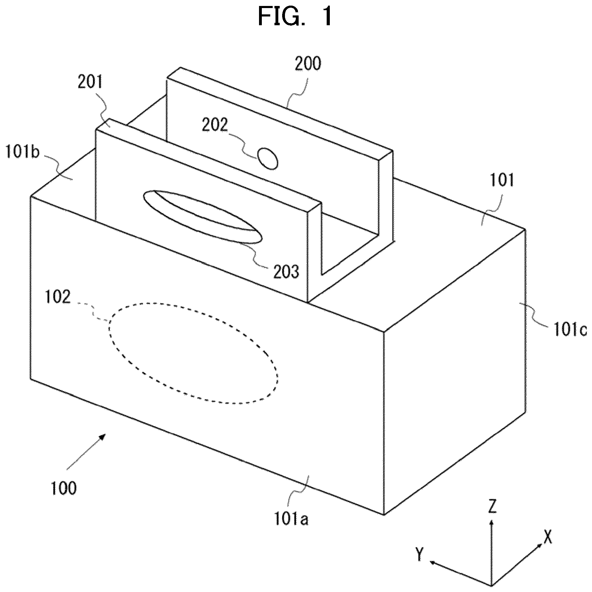

Fig. 1 is a view schematically illustrating an example of use of a measuring apparatus 100 to which the present invention is applied. In the example of use illustrated in Fig. 1, the measuring apparatus 100 includes an antenna 102 that transmits and receives a signal for acquiring biological information to and from a person to be measured in a casing 101 that is a rectangular parallelepiped exterior member. The measuring apparatus 100 performs so-called non-contact biological information sensing. A signal transmitted by the antenna 102 passes through a front surface 101a of the casing 101, reaches the person to be measured, is reflected from the person to be measured, and is detected by the antenna 102 that is a signal detector. Examples of the frequency of the signal transmitted from the antenna 102 to the person to be measured include frequencies in a frequency band of 30 GHz to 300 GHz used for a millimeter-wave radar, but frequencies in other frequency bands such as light, radio waves, sound waves, and ultrasonic waves may be adopted. Although the shape of the antenna 102 is schematically illustrated as an ellipse in Fig. 1, various shapes may be adopted as the shape of the antenna 102. In the following description, the measuring apparatus 100 includes the antenna 102, but a signal detector may be adopted in place of the antenna 102 in the measuring apparatus 100 according to the frequency of the signal to be used. A signal transmission method to be used may be a continuous wave such as frequency modulated continuous wave (FM-CW) or CW, or may be an intermittent wave. The antenna 102 may be formed of a transmission antenna and a reception antenna separately.

The measuring apparatus 100 has a sighting device 200 disposed on an upper surface 101b of the casing 101 for a user of the measuring apparatus 100, the person to be measured, or others to confirm the signal detection range (signal transmission range) of the antenna 102. The sighting device 200 is fixed to the upper surface 101b by a mounting member (not illustrated). The sighting device 200 includes a main body 201, and the main body 201 is provided with a visible mark 202 and a window portion 203. The material of the sighting device 200 is not particularly limited, but more preferably, the sighting device 200 is formed of a material having a low relative permittivity, such as plastic, which does not interfere with signals transmitted and received by the antenna 102 as much as possible. The size of the window portion 203 is determined based on the directivity of the antenna 102 such that a position is within the signal detection range of the antenna 102 as long as the mark 202 is visible through the window portion 203 from that position. The relationship between the range in which the mark 202 is visible through the window portion 203 and the signal detection range of the antenna 102 will be described later.

By providing such a sighting device 200 separately from the measuring apparatus 100 or integrally with the measuring apparatus 100, the user of the measuring apparatus 100 or the person to be measured can specify the signal detection range of the antenna 102 with a simple configuration. This can prevent the person to be measured from being out of the signal detection range of the antenna 102, improving the measurement accuracy of the measuring apparatus 100.

### <Description of Embodiments>

### (First Embodiment)

A first embodiment of the technique of the present disclosure will be described. First, in Fig. 1, XYZ axes orthogonal to each other are determined. In the embodiment, the casing 101 is a rectangular parallelepiped exterior member, and XYZ axes are determined such that a YZ plane is a plane parallel to the front surface 101a (and the back surface) of the casing 101, an XY plane is a plane parallel to the upper surface 101b (and the bottom surface) of the casing 101, and an XZ plane is a plane parallel to a side surface 101c of the casing 101.

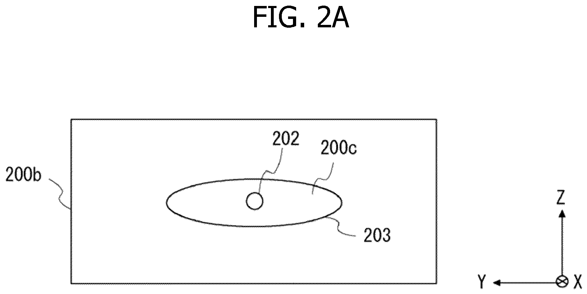

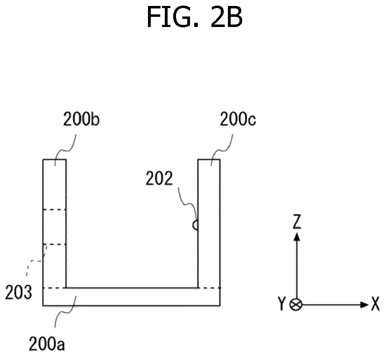

Fig. 2A illustrates a front view of the sighting device 200 fixed to the measuring apparatus 100, and Fig. 2B illustrates a side view of the sighting device 200. As illustrated in Fig. 2B, the sighting device 200 includes a flat plate-shaped base 200a parallel to the XY plane and two flat plate-shaped walls 200b, 200c extending from the base 200a in a direction parallel to the Z axis. The walls 200b, 200c are provided at separate positions so as to face each other. The wall 200b is provided with the window portion 203 penetrating the wall 200b in the X-axis direction, and the mark 202 is provided on the wall 200b side of the wall 200c. The mark 202 is formed using various paints as long as the user of the measuring apparatus 100 or the person to be measured can view the mark 200. Note that the mark 202 may be formed using a known technique, such as a projection or a recess formed on the surface of the wall 200c, a light-emitting diode (LED) lamp provided on the wall 200c, or a seal attached to the wall 200c, as long as the mark 202 are visible.

As illustrated in Fig. 2A, the window portion 203 is an elliptical window portion as viewed in the X-axis direction. As the shape of the window portion 203, various shapes may be appropriately adopted according to the directivity of the antenna 102. The mark 202 is formed on the wall 200c at a position where the mark 202 can be viewed so as to overlap with the intersection of the major and minor axes of the ellipse of the window portion 203 when the window portion 203 is viewed in the X-axis direction. Therefore, when the sighting device 200 is viewed in the X-axis direction from the front surface 101a side of the casing 101 of the measuring apparatus 100, the mark 202 is visible through the window portion 203. In Figs. 2A and 2B, the sighting device 200 is divided into the base 200a and the walls 200b, 200c for the sake of description, but the base 200a and the walls 200b, 200c may be integrally formed as illustrated in Fig. 1.

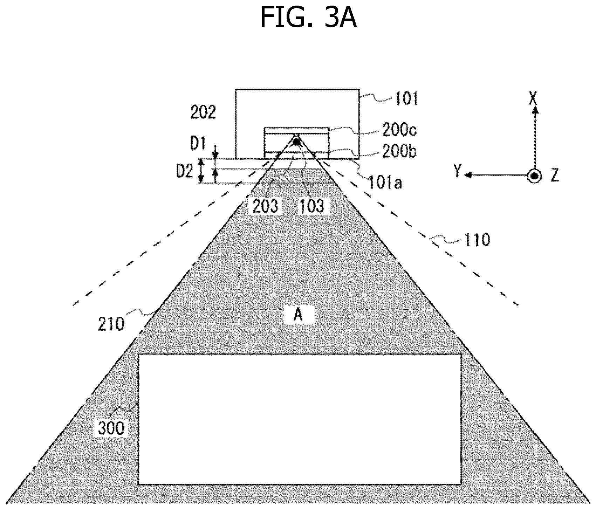

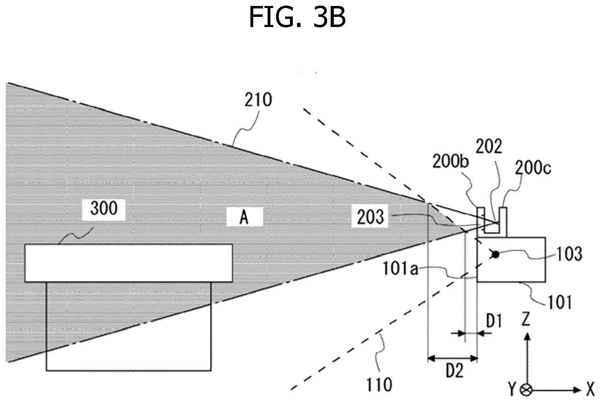

Next, the relationship between the range in which the mark 202 is visible through the window portion 203 and the signal detection range of the antenna 102 will be described with reference to Figs. 3A and 3B. Fig. 3A is a view schematically illustrating a range 210 in which the mark 202 is visible through the window portion 203 and a signal detection range 110 of the antenna 102 in a top view of the casing 101 of the measuring apparatus 100. Similarly, Fig. 3B is a view schematically illustrating the range 210 in which the mark 202 is visible through the window portion 203 and the signal detection range 110 of the antenna 102 in a side view of the casing 101 of the measuring apparatus 100.

The signal detection range 110 of the antenna 102 is determined based on the directivity of the antenna 102 with reference to a center 103 of the antenna 102 that receives a signal from the person to be measured. In the embodiment, as illustrated in Fig. 3A, it is assumed that the antenna 102 and the mark 202 are arranged such that the center 103 of the antenna 102 (in this example, the intersection of the major and minor axes of the ellipse of the antenna 102) and the mark 202 substantially overlap in the top view of the casing 101 of the measuring apparatus 100. In addition, as illustrated in Fig. 3B, it is assumed that the antenna 102 and the mark 202 are arranged such that the center 103 of the antenna 102 and the mark 202 are aligned vertically in the side view of the casing 101 of the measuring apparatus 100.

At this time, the range 210 in which the mark 202 is visible through the window portion 203 is formed on the front surface 101a side of the casing 101. The signal detection range 110 of the antenna 102 is formed on the front surface 101a side of the casing 101. Then, an area A is formed where at least a part of the range 210, in which the mark 202 is visible, is included in the signal detection range 110. Therefore, when the person to be measured is within that area A, a signal can be detected by the antenna 102. For example, as illustrated in Figs. 3A and 3B, when measurement is performed on a person to be measured on a bed 300 with the measuring apparatus 100, it is confirmed in advance that the user can view the mark 202 through the window portion 203 from the four corners of the bed 300 or from the top of the bed 300. This makes it possible to more reliably detect a signal from the person to be measured with the antenna 102 and obtain a more accurate measurement result than when measurement is performed without using the sighting device 200.

In addition, since the position of the center 103 of the antenna 102 differs from the position of the mark 202, in the example of Figs. 3A and 3B, the signal detection range 110 of the antenna 102 does not overlap with the range 210 in which the mark 202 is visible through the window portion 203 in the range of a distance D1 from the front surface 101a of the casing 101. Moreover, in the range of a distance D2 from the front surface 101a of the casing 101, there is an area where the signal detection range 110 of the antenna 102 and the range 210 in which the mark 202 is visible through the window portion 203 do not overlap. Thus, in the area within the distance D1 or distance D2 from the front surface 101a of the casing 101, there is a possibility that the signal cannot be detected by the antenna 102 even though the mark 202 can be confirmed through the window portion 203. Therefore, when the measuring apparatus 100 is used, setting a limit on the position of the person to be measured, such as ensuring the person to be measured at a distance greater than the distance D1 or distance D2 from the front surface 101a of the casing 101, can further improve the measurement accuracy of the measuring apparatus 100.

In the above example, the relative position between the mark 202 and the window portion 203 is a position at which the mark 202 can be viewed so as to overlap with the intersection of the major and minor axes of the ellipse of the window portion 203 when the window portion 203 is viewed in the X-axis direction. However, the relative position between the mark 202 and the window portion 203 may be appropriately changed according to the directivity of the antenna 102. Even if the center 103 of the antenna 102 and the mark 202 are arranged at positions shifted from each other in the top view of the casing 101 of the measuring apparatus 100, when the mark 202 can be viewed through the window portion 203 by changing the relative position between the mark 202 and the window portion 203 according to the directivity of the antenna 102, it is possible to detect a signal from the person to be measured with the antenna 102 and obtain a more accurate measurement result.

### (Second Embodiment)

Next, a second embodiment of the technique of the present disclosure will be described. In the measuring apparatus 100 according to the first embodiment, the sighting device 200 is configured separately from the measuring apparatus 100. However, the sighting device can also be configured integrally with the measuring apparatus.

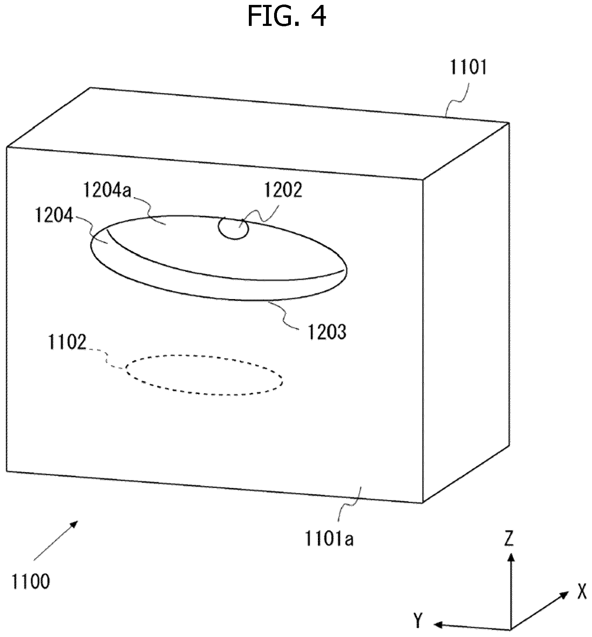

Fig. 4 illustrates an example of a measuring apparatus 1100 according to the embodiment. As illustrated in Fig. 4, in the measuring apparatus 1100, an antenna 1102 is disposed in a casing 1101 that is a rectangular parallelepiped exterior member. A recess 1204, where a part of a front surface 1101a is recessed in the X-axis direction, is formed in the front surface 1101a of the casing 1101. The recess 1204 includes an elliptical opening 1203 similar to the window portion 203 of the first embodiment and a bottom surface 1204a having a shape corresponding to the shape of the opening 1203. A mark 1202 corresponding to the mark 202 of the first embodiment is formed on a bottom 1204a that is a part of the inner surface of the recess 1204.

In this embodiment, similarly to the first embodiment, it is assumed that the antenna 1102 and the mark 1202 are arranged such that a center 1103 of the antenna 1102 and the mark 1202 substantially overlap in a top view of the casing 1101 of the measuring apparatus 1100, and such that the center 103 of the antenna 102 and the mark 202 are aligned vertically in a side view of the casing 1101 of the measuring apparatus 1100.

In this embodiment, the opening 1203 of the recess 1204 corresponds to the window portion 203 of the first embodiment, the mark 1202 of the recess 1204 corresponds to the mark 202 of the first embodiment, and the recess 1204 including the opening 1203 and the mark 1202 functions as a sighting device corresponding to the sighting device 200 of the first embodiment. Thus, it is possible to determine whether a user of the measuring apparatus 1100 or a person to be measured is within the signal detection range of the antenna 1102, based on whether the mark 1202 is visible through the opening 1203 of the recess 1204.

Therefore, in this embodiment as well, similarly to the example of the first embodiment, when measurement is performed on the person to be measured with the measuring apparatus 1100, it is confirmed in advance that the user can view the mark 1202 through the opening 1203 of the recess 1204. This makes it possible to more reliably detect a signal from the person to be measured with the antenna 1102 and obtain a more accurate measurement result than when the measurement is performed without using the configuration like the recess 1204.

In addition, in the measuring apparatus 1100 of this embodiment, since the recess 1204 functioning as a sighting device is formed in a part of the front surface 1101a of the casing 1101, the mark 1202 can be disposed at a position closer to the center of the antenna 1102 compared to the first embodiment. Thus, the area where the range in which the mark is visible and the signal detection range of the antenna overlap is larger than in the case of the first embodiment. As a result, it is possible to more reliably perform signal detection on the person to be measured with the antenna 1102 and to further improve the measurement accuracy of the measuring apparatus 1100.

In the above example, the recess 1204 is formed as an elliptic columnar cavity, but the inner surface shape of the recess 1204 is not limited thereto. For example, the inner surface of the recess 1204 may be formed of a paraboloid surface, and the mark 1202 may be provided on a part of the paraboloid surface.

### <Others>

The above embodiments are merely illustrative examples of the configurations of the present invention. The present invention is not limited to the specific forms described above, and various modifications can be made within the scope of the technical idea. In the following, modifications of the above embodiments will be described. Note that the embodiments described above and the modifications described below can be appropriately combined and implemented.

### (Modification 1)

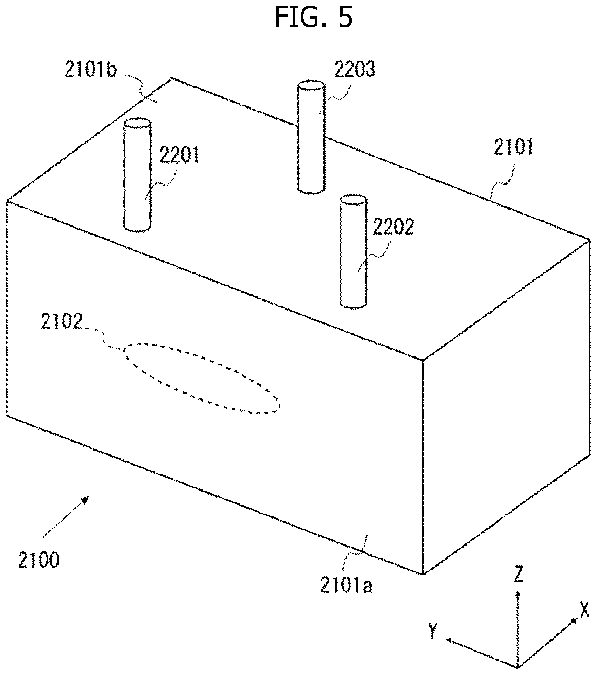

A measuring apparatus 2100 according to Modification 1 will be described below. Fig. 5 illustrates an example of the measuring apparatus 2100 according to the present modification. As illustrated in Fig. 5, in the measuring apparatus 2100, an antenna 2102 is disposed in a casing 2101 that is a rectangular parallelepiped exterior member. In the measuring apparatus 2100 as well, similarly to the above embodiments, a signal transmitted by the antenna 2102 passes through a front surface 2101a of the casing 2101, reaches a person to be measured, is reflected by the person to be measured, and is detected by the antenna 2102.

On an upper surface 2101b that is the outer surface of the casing 2101, three columnar rod members 2201, 2202, 2203 extending in the Z-axis direction from the upper surface 2101b are provided. Note that the rod members 2201 to 2203 may be removably provided on the casing 2101, or may be integrally provided with the casing 2101. On the upper surface 2101b, two rod members 2201, 2202 of the three rod members 2201 to 2203 are disposed closer to the front surface 2101a than the remaining rod member 2203. In the present modification, the three rod members 2201 to 2203 function as a sighting device corresponding to the sighting device 200 of the first embodiment.

In a top view of the casing 2101, the rod member 2203 is disposed at a position substantially overlapping with the center of the antenna 2102, similarly to the marks 202, 1202 described above. Further, in the top view of the casing 2101, the positions of the rod members 2201, 2202 are set such that the range in which the rod member 2203 is visible between the rod members 2201 and 2202 when viewed in the X-axis direction from the front surface 2101a side of the casing 2101 is included in the signal detection range of the antenna 2102. As a result, the rod members 2201, 2202 determine the viewing range of the rod member 2203 to specify the position as being within the signal detection range of the antenna 2102 in the azimuth angle (the angle of rotation about the Z axis in the XY plane) direction of the antenna 2102.

Thus, in the present modification as well, similarly to the area A illustrated in Fig. 3A in the first embodiment, on the front surface 2101a side of the casing 2101, it is possible to form an area where the range in which the rod member 2203 is visible between the rod members 2201 and 2202 is included in the signal detection range of the antenna 2102. Therefore, when the person to be measured is within that area, the signal can be detected by the antenna 2102.

According to the present modification, the measurement accuracy of the measuring apparatus can be expected to be improved by placing the person to be measured within the signal detection range of the antenna, using a simpler configuration than the sighting device 200 of the first embodiment.

### (Modification 2)

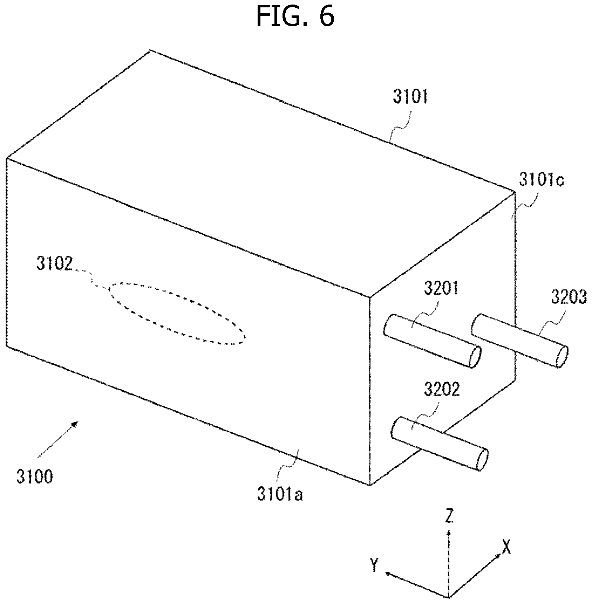

Next, a measuring apparatus 3100 according to Modification 2 will be described. Fig. 6 illustrates an example of the measuring apparatus 3100 according to the present modification. As illustrated in Fig. 6, in the measuring apparatus 3100, an antenna 3102 is disposed in a casing 3101 that is a rectangular parallelepiped exterior member. In the measuring apparatus 3100 as well, similarly to the above embodiments and modification, a signal transmitted by the antenna 3102 passes through a front surface 3101a of the casing 3101, reaches a person to be measured, is reflected by the person to be measured, and is detected by the antenna 3102.

On a side surface 3101c that is the outer surface of the casing 3101, three columnar rod members 3201, 3202, 3203 extending in the Y-axis direction from the side surface 3101c are provided. Note that the rod members 3201 to 3203 may be removably provided on the casing 3101, or may be integrally provided with the casing 3101. On the side surface 3101c, two rod members 3201, 3202 of the three rod members 3201 to 3203 are disposed closer to the front surface 3101a than the remaining rod member 3203. In the present modification, the three rod members 3201 to 3203 function as a sighting device corresponding to the sighting device 200 of the first embodiment.

In a side view of the casing 3101, the rod member 3203 is disposed at a position substantially overlapping with the center of the antenna 3102. In the side view of the casing 3101, the positions of the rod members 3201, 3202 are set such that the range in which the rod member 3203 is visible between the rod members 3201 and 3202 when viewed in the X-axis direction from the front surface 3101a side of the casing 3101 is included in the signal detection range of the antenna 3102. As a result, the rod members 3201, 3202 determine the viewing range of the rod member 3203 to specify the position as being within the signal detection range of the antenna 3102 in the elevation angle (the angle of rotation about the Y axis in the XZ plane) direction of the antenna 3102.

Thus, in the present modification as well, similarly to the area A illustrated in Fig. 3B in the first embodiment, on the front surface 3101a side of the casing 3101, it is possible to form an area where the range in which the rod member 3203 is visible between the rod members 3201 and 3202 is included in the signal detection range of the antenna 3102. Therefore, when the person to be measured is within that area, the signal can be detected by the antenna 3102.

According to the present modification, similarly to Modification 1, the measurement accuracy of the measuring apparatus can be expected to be improved by placing the person to be measured within the signal detection range of the antenna, using a simpler configuration than the sighting device 200 of the first embodiment.

### (Modification 3)

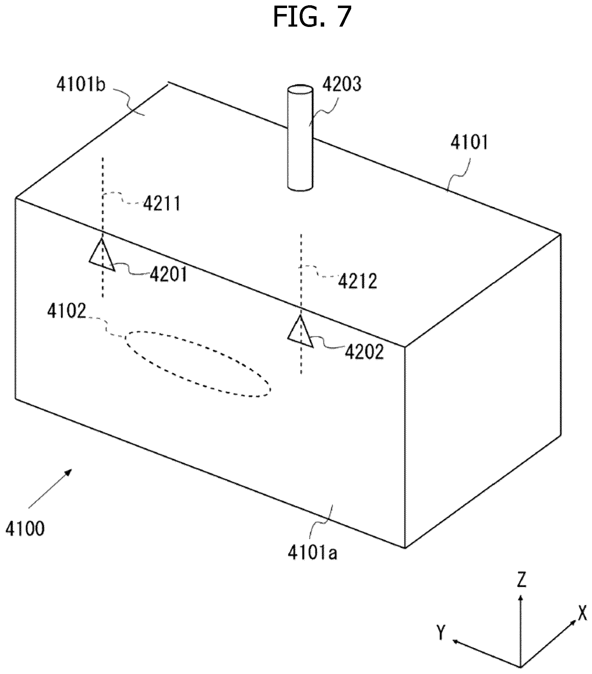

Next, a measuring apparatus 4100 according to Modification 3 will be described. Fig. 7 illustrates an example of the measuring apparatus 4100 according to the present modification. As illustrated in Fig. 7, in the measuring apparatus 4100, an antenna 4102 is disposed in a casing 4101 that is a rectangular parallelepiped exterior member. In the measuring apparatus 4100 as well, similarly to the above embodiments and modifications, a signal transmitted by the antenna 4102 passes through a front surface 4101a of the casing 4101, reaches a person to be measured, is reflected by the person to be measured, and is detected by the antenna 4102.

Further, two marks 4201, 4202 are provided on the front surface 4101a of the casing 4101. The marks 4201, 4202 are provided near the upper end of the front surface 4101a. On an upper surface 4101b of the casing 4101, a columnar rod member 4203 extending in the Z-axis direction from the upper surface 4101b is provided. The marks 4201, 4202 are indicators in place of the rod members 2201, 2202 of Modification 1, and are indicators that define virtual boundary lines 4211, 4212 in the azimuth angle (the angle of rotation about the Z axis in the XY plane) direction of the antenna 4102 when the rod member 4203 is viewed to confirm the signal detection range of the antenna 4120. The virtual boundary lines 4211, 4212 can be regarded as lines extending in the Z-axis direction through the marks 4201, 4202. Note that any shape may be adopted as the shapes of the marks 4201, 4202 as long as the marks 4201, 4202 are visible. In the present modification, the two marks 4201, 4202 and the rod member 4203 function as a sighting device corresponding to the sighting device 200 of the first embodiment.

In a top view of the casing 4101, the rod member 4203 is disposed at a position substantially overlapping with the center of the antenna 4102, similarly to the rod member 2203 described above. Further, in the top view of the casing 4101, the positions of the marks 4201, 4202 are set such that the range in which the rod member 4203 is visible between the virtual boundary lines 4211 and 4212, defined by the marks 4201, 4202, when viewed in the X-axis direction from the front surface 4101a side of the casing 4101 is included in the signal detection range in the azimuth angle direction of the antenna 4102. Therefore, the virtual boundary lines 4211, 4212, defined by the marks 4201, 4202, determine the viewing range of the rod member 4203 to specify the position as being within the signal detection range of the antenna 4102 in the azimuth angle (the angle of rotation about the Z axis in the XY plane) direction of the antenna 4102. Accordingly, in the present modification as well, similarly to Modification 1, on the front surface 4101a side of the casing 4101, it is possible to form an area where the range in which the rod member 4203 is visible between the virtual boundary lines 4211 and 4212, defined by the marks 4201, 4202, is included in the signal detection range of the antenna 4102. Therefore, when the person to be measured is within that area, the signal can be detected by the antenna 4102.

According to the present modification, the measurement accuracy of the measuring apparatus can be expected to be improved by placing the person to be measured within the signal detection range of the antenna, using a simpler configuration than the sighting device 200 of the first embodiment.

### (Modification 4)

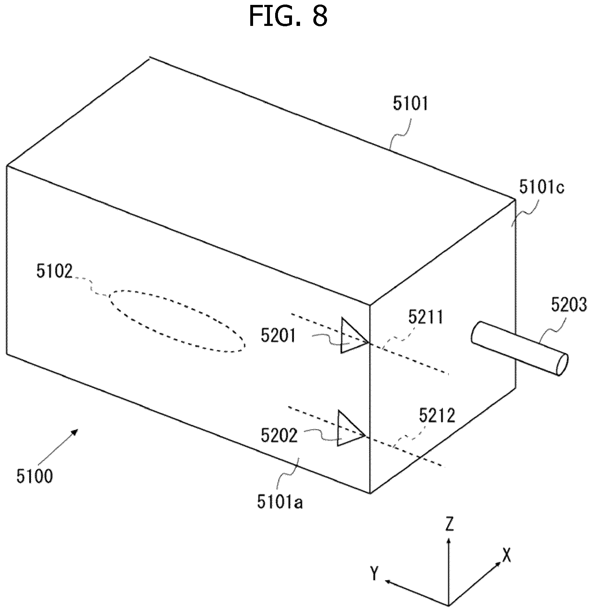

Next, a measuring apparatus 5100 according to Modification 4 will be described. Fig. 8 illustrates an example of the measuring apparatus 5100 according to the present modification. As illustrated in Fig. 8, in the measuring apparatus 5100, an antenna 5102 is disposed in a casing 5101 that is a rectangular parallelepiped exterior member. In the measuring apparatus 5100 as well, similarly to the above embodiments and modifications, a signal transmitted by the antenna 5102 passes through a front surface 5101a of the casing 5101, reaches a person to be measured, is reflected by the person to be measured, and is detected by the antenna 5102.

Two marks 5201, 5202 are provided on the front surface 5101a of the casing 5101. The marks 5201, 5202 are provided near one end (the right end in the drawing) of the front surface 5101a in the horizontal direction. On a side surface 5101c of the casing 5101, a columnar rod member 5203 extending in the Y-axis direction from the side surface 5101c is provided. The marks 5201, 5202 are indicators similar to the marks 4201, 4202 of Modification 3, and are indicators that define virtual boundary lines 5211, 5212 in the elevation angle (the angle of rotation about the Y axis in the XZ plane) direction when the rod member 5203 is viewed to confirm the signal detection range of the antenna 5102. The virtual boundary lines 5211, 5212 can be regarded as lines extending in the Y-axis direction through the marks 5201, 5202. Note that any shape may be adopted as the shapes of the marks 5201, 5202 as long as the marks 5201, 5202 are visible. In the present modification, the two marks 5201, 5202 and the rod member 5203 function as a sighting device corresponding to the sighting device 200 of the first embodiment.

In a side view of the casing 5101, the rod member 5203 is disposed at a position substantially overlapping with the center of the antenna 5102, similarly to the rod member 3203 described above. In the side view of the casing 5101, the positions of the marks 5201, 5202 are set such that the range in which the rod member 5203 is visible between the virtual boundary lines 5211 and 5212, defined by the marks 5201, 5202, when viewed in the X-axis direction from the front surface 5101a side of the casing 5101 is included in the signal detection range of the antenna 5102. Therefore, the virtual boundary lines 5211, 5212, defined by the marks 5201, 4502, determine the viewing range of the rod member 5203 to specify the position as being within the signal detection range of the antenna 5102 in the elevation angle (the angle of rotation about the Y axis in the XZ plane) direction of the antenna 5102. Accordingly, in the present modification as well, similarly to Modification 3, on the front surface 5101a side of the casing 5101, it is possible to form an area where the range in which the rod member 5203 is visible between the virtual boundary lines 5211 and 5212, defined by the marks 5201, 5202, is included in the signal detection range of the antenna 5102. Therefore, when the person to be measured is within that area, the signal can be detected by the antenna 5102.

According to the present modification, the measurement accuracy of the measuring apparatus can be expected to be improved by placing the person to be measured within the signal detection range of the antenna, using a simpler configuration than the sighting device 200 of the first embodiment.

### (Modification 5)

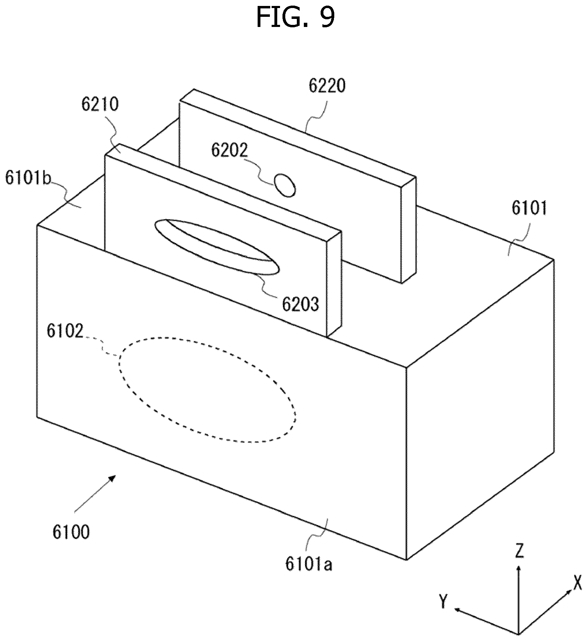

Next, a measuring apparatus 6100 according to Modification 5 will be described. Fig. 9 illustrates an example of the measuring apparatus 6100 according to the present modification. As illustrated in Fig. 9, in the measuring apparatus 6100, an antenna 6102 is disposed in a casing 6101 that is a rectangular parallelepiped exterior member. In the measuring apparatus 6100 as well, similarly to the above embodiments, a signal transmitted by the antenna 6102 passes through a front surface 6101a of the casing 6101, reaches a person to be measured, is reflected by the person to be measured, and is detected by the antenna 6102.

In the present modification, the walls 200b, 200c constituting the sighting device 200 of the first embodiment are configured as separate walls 6210, 6220. A window portion 6203 corresponding to the window portion 203 of the first embodiment is formed in the wall 6210, and a mark 6202 corresponding to the mark 202 of the first embodiment is formed of the wall 6220. The walls 6210, 6220 are fixed to an upper surface 6101b of the casing 6101 using respective mounting member (not illustrated). Note that the mounting member can be implemented using a known technique, and therefore a detailed description thereof will be omitted. In the present modification, the two walls 6210, 6220 function as a sighting device corresponding to the sighting device 200 of the first embodiment.

When the walls 6210, 6220 are fixed to the upper surface 6101b, the relationship among the mark 6202, the window portion 6203, and the center of the antenna 6102 corresponds to the relationship among the mark 202, the window portion 203, and the center of the antenna 102 in the first embodiment. Thus, on the front surface 6101a side of the casing 6101, an area is formed where the range in which the mark 6202 is visible through the window portion 6203 is included in the signal detection range of the antenna 6102. Therefore, when the person to be measured is within that area, the signal can be detected by the antenna 6102.

According to the present modification, the measurement accuracy of the measuring apparatus can be expected to be improved by placing the person to be measured within the signal detection range of the antenna, using a configuration similar to the sighting device 200 of the first embodiment.

### (Modification 6)

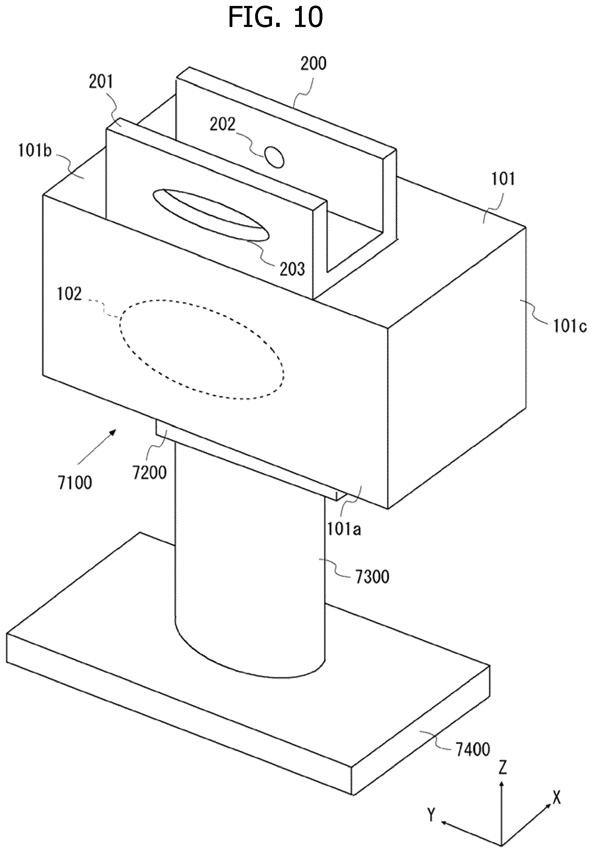

Next, a measuring apparatus 7100 according to Modification 6 will be described. Fig. 10 illustrates an example of the measuring apparatus 7100 according to the present modification. As illustrated in Fig. 10, the measuring apparatus 7100 has a configuration similar to the measuring apparatus 100 and the sighting device 200 in the first embodiment. Therefore, in the following description, components similar to those of the first embodiment are denoted by the same reference numerals, and a detailed description thereof will be omitted.

In the present modification, the measuring apparatus 7100 includes a flat plate-shaped pedestal 7400 and a cylindrical support portion 7300 for supporting the casing 101 to which the sighting device 200 is fixed. Moreover, the measuring apparatus 7100 includes an adjuster 7200 for changing the orientation of the casing 101, the orientation of the sighting device 200 fixed to the casing 101 in the azimuth angle (the angle of rotation about the Z axis in the XY plane) direction and the elevation angle (the angle of rotation about the Y axis in the XZ plane) direction of the antenna 102. As an example, the adjuster 7200 rotates the casing 101 about the Y axis and the Z axis. The rotation mechanism of the adjuster 7200 can be implemented by using a known technique, and therefore a detailed description thereof will be omitted here. The adjuster 7200 may be configured to change the orientation of the sighting device 200 in at least one of the azimuth angle direction or the elevation angle direction of the antenna 102.

Thus, since the direction of the sighting device 200 can be changed in accordance with the position of the person to be measured at the time of measurement, the measurement accuracy of the measuring apparatus can be expected to be improved by changing the signal detection range of the antenna according to the use environment of the measuring apparatus 7100 and by placing the person to be measured within the signal detection range of the antenna.

### (Modification 7)

Next, a measuring apparatus according to Modification 7 will be described. In the present modification, components similar to those of the above embodiments are denoted by the same reference numerals, and a detailed description thereof will be omitted. In the present modification, as an example, an infrared sensor is provided instead of the mark 202 in the sighting device 200 of the first embodiment described above. The Wall 200b in which the window portion 203 is formed is made of a material that hardly transmits or substantially blocks infrared light used for the infrared sensor. By using the sighting device 200 configured as described above, the measuring apparatus 100 can detect a person within the signal detection range of the antenna 102 with the infrared sensor and notify the person with a lamp, a speaker, or the like (not illustrated). This enables a user of the measuring apparatus 100, a person to be measured, or others to confirm whether he or she is within the signal detection range of the antenna 102 by using the sighting device 200. As a result, in the present modification as well, it is possible to more reliably detect a signal from the person to be measured with the antenna 102 and obtain a more accurate measurement result than when measurement is performed without using the sighting device 200.

### <Supplementary Note 1>

A sighting device (200) used in a measuring apparatus (100) including a signal detector (102) that detects a signal related to biological information reflected from a person to be measured, the sighting device including:
a visible indicator (202); and
a viewing range specifier (203) configured to specify a viewing range of the indicator,
in which a relative position between the indicator and the viewing range specifier is determined such that the viewing range of the indicator specified by the viewing range specifier forms an area included in a detection range of the signal by the signal detector.

### DESCRIPTION OF SYMBOLS

- 100: measuring apparatus
- 102: antenna
- 200: sighting device
- 202: mark
- 203: window portion

## Claims

1. A sighting device used in a measuring apparatus including a signal detector that detects a signal related to biological information reflected from a person to be measured, the sighting device comprising:
a visible indicator; and
a viewing range specifier configured to specify a viewing range of the indicator,
wherein a relative position between the indicator and the viewing range specifier is determined such that the viewing range of the indicator specified by the viewing range specifier forms an area included in a detection range of the signal by the signal detector.

2. The sighting device according to claim 1, wherein
the indicator is formed on an inner surface of a recess formed in an exterior member of the measuring apparatus, and
the viewing range specifier is an opening of the recess.

3. The sighting device according to claim 1, further comprising two walls facing each other, wherein
the indicator is formed on one wall of the two walls, and
the viewing range specifier is a window portion penetrating another wall of the two walls.

4. The sighting device according to claim 1, wherein the viewing range specifier is two rod members extending from an outer surface of an exterior member of the measuring apparatus, the two rod members being disposed at positions for specifying a viewing range of the indicator in at least one of an azimuth angle direction or an elevation angle direction as viewed from the signal detector.

5. The sighting device according to claim 1, wherein the viewing range specifier is two indicators visible from an outside of the measuring apparatus, the two indicators being disposed at positions for specifying a viewing range of the indicator in at least one of an azimuth angle direction or an elevation angle direction as viewed from the signal detector.

6. A measuring apparatus comprising the sighting device according to any one of claims 1 to 5 and the signal detector.

7. The measuring apparatus according to claim 6, further comprising an adjuster configured to change a direction of the sighting device in at least one of an azimuth angle direction or an elevation angle direction as viewed from the signal detector.
